# EUROPEAN PATENT SPECIFICATION

(11) **EP 2 400 031 B1**
(45) Date of publication and mention of the grant of the patent: **10.08.2016**
(21) Application number: 11170253.6
(22) Date of filing: 16.06.2011
(51) Int. Cl.: C12Q 1/00, G01N 33/66, G01N 33/72

(54) **Method for measuring plasma glucose**
Verfahren zum Messen von Plasma-Glucose
Procédé pour mesurer le glucose plasmatique

(30) Priority: 23.06.2010 JP 2010142173; 08.06.2011 JP 2011127853
(43) Date of publication of application: 28.12.2011
(73) Proprietor: ARKRAY, Inc., Minami-ku Kyoto-shi Kyoto 601-8045 (JP)
(72) Inventor: Takagi, Takeshi, Kyoto-shi Kyoto 602-0008 (JP); Okumura, Koji, Kyoto-shi Kyoto 602-0008 (JP)
(74) Representative: Hall, Matthew Benjamin

(56) References cited:
- EP-A1- 1 806 580
- US-A1- 2008 300 170
- US-A1- 2009 184 004

## Description

This invention relates to a method and device for measuring plasma glucose to measure a plasma glucose concentration. This invention also relates to a system for measuring plasma glucose and a blood analysis system.

The measurement of a plasma glucose concentration is carried out for a screening test and follow-up for diabetes.

In the measurement of a plasma glucose concentration, an enzyme electrode method is widely used which detects, with an electrode, an oxidation-reduction current of a product caused by an enzyme reaction of glucose and a glucose oxidoreductase to convert the detected current into a glucose concentration. As the enzyme electrode method, an equilibrium point method and a differentiation are known (Japanese Examined Patent Application Publication No. H07-37991).

In the blood components analysis, it is ideal to avoid centrifugations for removing hemocyte (blood cell) components in blood in terms of improving analysis speed when preparing a sample provided for a measurement. Therefore, seen from this standpoint, it is required that a measurement sample should be prepared using blood (whole blood) including hemocytes as a specimen when a plasma glucose concentration is measured with the enzyme electrode method.

However, a measurement error problem occurs which is derived from individual differences between ratios of hemocytes (hematocrit values) in blood when a measurement sample is prepared using whole blood as a specimen to measure a glucose concentration in the measurement sample with the enzyme electrode method (Japanese Examined Patent Application Publication No. H07-37991).

For this problem, the technology has been developed, which makes a measurement error small derived from an individual difference in a hemocyte concentration in blood by estimating a ratio of hemocytes in blood from calculated results of glucose concentrations obtained with the equilibrium point method and the differentiation respectively (Japanese Examined Patent Application Publication No. H07-37991, Japanese Unexamined Patent Application Publication No. H09-33533, and Japanese Unexamined Patent Application Publication No. H09-318634).

For this problem, a technology is also reported, which uses a hemolyzed sample prepared by preliminarily breaking hemocyte components in blood with ultrasound in measurement (Japanese Unexamined Patent Application Publication No. S60-192249).

For a technology using a hemolyzed sample in measurement, a method is also reported, which corrects an influence of a hematocrit value to measure glucose by hemolyzing red hemocytes, reacting hemoglobin flowing out the of red hemocytes and a mediator, and detecting a current caused by this reaction when an oxidation-reduction current of glucose is detected (Japanese Unexamined Patent Application Publication No. 2005-114359).

On the other hand, as a device for measuring plasma glucose for measuring a plasma glucose concentration, a blood analysis device is known, which is configured to carry out measurement of a glucose concentration and a glycohemoglobin concentration with sampling blood at once (WO 2008/035748).

Technologies described in patent literatures 1 to 3 are simple in terms of processing and calculating numeric values obtained by a conventional method for measuring a glucose concentration while it cannot eliminate sufficient error causes which output waveform noises and the like of a measurement device obtains a calculated result.

A measurement object in the technologies described in the patent literatures 4 and 5 is a glucose concentration in a hemolyzed sample, not a plasma glucose concentration itself. Thus, there has still been a problem on an error between a glucose concentration (measured value) in a hemolyzed sample and an actual plasma glucose concentration.

A blood analysis device described in the patent literature 6 has not had enough measurement accuracy of a plasma glucose concentration yet.

US-A-2008/0300170 discloses identification of biological markers associated with an increased risk of developing diabetes. US-A-2009/0184004 discloses a method for correcting a measurement of glucose concentration in a blood sample with a high hematocrit level by determining the hematocrit level, determining a correction factor and correcting the initial measurement. EP-A1-806508 discloses a method for measuring glucose in a blood sample independently of hematocrit by relating the concentration to a concentration calculated using a differential method and a concentration calculated using an equilibrium point method.

An aim of this invention is to provide a conventional technology for increasing measurement accuracy of a plasma glucose concentration.

Further, another aim of this invention is to provide a technology for increasing measurement accuracy of a plasma glucose concentration with maintaining or improving a conventional measurement speed of a plasma glucose concentration.

These inventors have continued to develop the conventional technology for increasing the measurement accuracy of the plasma glucose concentration on the assumption that blood including hemocytes is used as a specimen to speed up an analysis by omitting a pretreatment operation on blood, such as a centrifugation.

In this process, as a result of many examinations on errors between glucose concentrations (measured values) obtained from measurement with the foregoing hemolyzed samples and actual plasma glucose concentrations, these inventors found out that one of the causes is based on a difference between a liquid content ratio (water content ratio) of plasma and a liquid content ratio of hemocytes. The inventors then found out that they can obtain a value extremely close to the actual plasma glucose concentration by using a hemocyte/plasma ratio in the blood, liquid content ratio of hemocytes and liquid content ratio of plasma.

In addition, hereinafter the term of "ratio" is used to refer to volume ratio (v/v) in this description.

According to a first aspect, the present invention provides a method for measuring plasma glucose (hereinafter referred to as "the method for measuring plasma glucose of this invention") comprising calculating a plasma glucose concentration from a whole blood glucose concentration measured with a measurement sample prepared by hemolyzing hemocytes in blood, wherein the calculation is based on a hemocyte/plasma ratio in the blood, liquid content ratio of hemocytes and liquid content ratio of plasma in the blood, the method comprising a step of measuring the glucose concentration in whole blood with a measurement sample prepared by hemolyzing hemocytes in blood, a step of calculating a liquid content ratio of whole blood based on the hemocyte/plasma ratio in the blood, the liquid content ratio of hemocytes and the liquid content ratio of plasma, and a step of calculating plasma glucose concentration from the glucose concentration in whole blood, based on the calculated liquid content ratio of whole blood, by multiplying the whole blood glucose concentration with a multiplying factor of the liquid content ratio of plasma divided by the liquid content ratio of whole blood.

The method for measuring plasma glucose of this invention allows for measurement of the plasma glucose concentration with accuracy, and allows for exact measurement and calculation.

Preferably, the method for measuring plasma glucose of this invention further includes a step of measuring a hemoglobin concentration in whole blood with the measurement sample to calculate a hemocyte/plasma ratio of blood.

This method can calculate the hemocyte/plasma ratio in blood with accuracy and allows for exact measurement of the plasma glucose concentration.

In the method for measuring plasma glucose of this invention, the hemoglobin concentration is preferably measured with an absorptiometry.

This method can measure the hemoglobin concentration in the measurement sample with accuracy and allows for exact calculation of the hemocyte/plasma ratio.

Preferably, the method for measuring plasma glucose of this invention further comprise a sample preparation step of adding and stirring blood in diluent containing a hemolytic agent.

This method can dilute blood and hemolyze hemocytes together, and speed up an analysis. Stirring can also shorten time to complete from adding blood to hemolyzing hemocytes, and speed up the analysis.

In the method for measuring plasma glucose of this invention, the step of measuring whole blood glucose is preferably measured by a so-called enzyme electrode method of detecting, which detect an oxidation-reduction current of an enzyme reaction product of glucose and a glucose oxidoreductase using an electrode, or by so-called enzymatic colorimetric method of colorimetrically, which quantitate a reaction product of an enzyme reaction product of glucose and a chromogen.

This method can rapidly measure the glucose concentration in the measurement sample, whereby improving analysis speed.

Preferably, the method for measuring plasma glucose of this invention further comprise a correction step of correcting an influence which an enzymatic reaction rate of an unlysed blood sample before finishing hemolysis of all hemocytes in the sample preparation step exerts on an enzymatic reaction rate of the measurement sample.

This method can correct a small delay until a stable current value occurs or a stable amount of dye is obtained, which small delay is caused when the glucose concentration is tried to be measured rapidly with the measurement sample whose hemocytes is hemolyzed, and further improve measurement accuracy of the plasma glucose concentration.

According to a second aspect, the present invention provides a device for measuring plasma glucose (hereinafter referred to as "the device for measuring plasma glucose of this invention") comprising a section configured to measure whole blood glucose, which measures a glucose concentration in whole blood with a measurement sample prepared by hemolyzing hemocytes in blood, a section configured to calculate plasma glucose, which calculates a plasma glucose concentration from the glucose concentration in whole blood based on a hemocyte/plasma ratio in the blood, the liquid content ratio of hemocytes and the liquid content ratio of plasma, and a section configured to calculate a liquid content ratio of whole blood, which calculates a liquid content ratio of whole blood from the hemocyte/plasma ratio in the blood, predetermined liquid content ratio in hemocytes and predetermined liquid content ratio of plasma, wherein the section for calculating plasma glucose calculates a plasma glucose concentration by multiplying a measured value of whole blood glucose concentration with a multiplying factor of the liquid content ratio of plasma divided by the calculated value of the liquid content ratio of whole blood and outputs such calculated value of the plasma glucose concentration.

The device for measuring plasma glucose of this invention allows for measurement of the plasma glucose concentration with accuracy using blood as a specimen. This configuration allows for exact calculation of the plasma glucose concentration.

The preferred mode of the device for measuring plasma glucose of this invention further comprises a section for calculating a hemocyte/plasma ratio, which measures a whole blood hemoglobin concentration of the measurement sample and calculates a hemocyte/plasma ratio in blood from the hemoglobin concentration.

This configuration can calculate the hemocyte/plasma ratio in blood with accuracy, and allows for exact calculation of the plasma glucose concentration.

In a preferred mode of the device for measuring plasma glucose of this invention, the section for calculating a hemocytes/plasma ratio has an absorption spectrometer for measuring the hemoglobin concentration.

This configuration can measure the hemoglobin concentration in the measurement sample with accuracy, and allows for exact calculation of a hemocyte/plasma ratio.

In a preferred mode of the device for measuring plasma glucose of this invention, a sample preparation means for preparing the measurement sample in which hemocytes in blood are hemolyzed is comprised.

This configuration can prepare and start a reaction of glucose and a glucose oxidoreductase and a voltage application to an electrode in parallel with a sample preparation, and rapidly obtain an intended current value to improve analysis speed.

In a preferred mode of the device for measuring plasma glucose of this invention, the section for measuring whole blood glucose has a sensor means including an enzyme-immobilized layer with immobilized glucose oxidoreductase and an electrode for detecting an oxidation-reduction current of an enzyme reaction product of glucose and a glucose oxidoreductase.

This configuration can start the reaction of glucose and the glucose oxidoreductase and the voltage application to the electrode in parallel with the sample preparation, and rapidly obtain the intended current value to improve the analysis speed.

According to a third aspect, the present invention provides a program for measuring plasma glucose (hereinafter referred to as "the program for measuring plasma glucose of this invention") to make a computer work as: a section for calculating a whole blood glucose concentration, which calculates a glucose concentration in whole blood based on a value correlated with a glucose concentration of a measurement sample whose hemocytes in blood are hemolyzed, a section for calculating plasma glucose, which calculates a plasma glucose concentration from a measured value of blood glucose concentration based on a hemocyte/plasma ratio in the blood, predetermined liquid content ratio of hemocytes and predetermined liquid content ratio of plasma and outputs such calculated value of the plasma glucose concentration, and a section for calculating a liquid content ratio of whole blood, which calculates a liquid content ratio of whole blood from the hemocyte/plasma ratio in the blood, the predetermined liquid content ratio of hemocytes and the predetermined liquid content ratio of plasma, wherein the section for calculating plasma glucose further calculates a plasma glucose concentration by multiplying a measured value of whole blood glucose concentration with a multiplying factor of the liquid content ratio of plasma divided by a calculated value of the liquid content ratio of whole blood and outputs such resultant calculated value of the plasma glucose concentration.

It allows for plasma glucose concentration measurement with accuracy using blood as a specimen to mount the program for measuring plasma glucose of this invention on a computer of a device for measuring plasma glucose.

Preferably, the program for measuring plasma glucose of this invention is further to make a computer work as a section for calculating a hemocyte/plasma ratio, which calculates a hemoglobin concentration in whole blood based on a value correlated with a hemoglobin concentration of the measurement sample and calculating a hemocyte/plasma ratio of blood from the hemoglobin concentration.

This invention also provides a computer-readable recording medium for recording these programs.

This invention provides a system for giving a plurality of devices a function of each section of the device for measuring plasma glucose of this invention as described above to realize the method for measuring plasma glucose of this invention as described above among respective devices.

According to a fourth aspect, the present invention provides a system for measuring plasma glucose (hereinafter referred to as "the system for measuring plasma glucose of this invention") which comprises a device configured to detect whole blood glucose concentration, which detects a value correlated with a glucose concentration of a measurement sample prepared by hemolyzing hemocytes in blood, a device configured to detect a hemocyte/plasma ratio, which detects a value correlated with a hemocyte/plasma ratio of the blood, and a calculating device configured to calculate a plasma glucose concentration based on each detection value obtained from the devices for detecting whole blood glucose concentration and the hemocyte/plasma ratio, wherein the calculating device has a section configured to calculate a whole blood glucose concentration, which calculates a glucose concentration in whole blood based on a detection value obtained from the device for detecting glucose concentration, a section configured to calculate plasma glucose concentration, which calculates a plasma glucose concentration from the glucose concentration value in whole blood calculated at the section for calculating the whole blood glucose concentration, based on a detection value obtained from the device for detecting the hemocytes/plasma ratio, predetermined liquid content ratio in hemocytes and predetermined liquid content ratio of plasma and outputs such calculated value of the plasma glucose concentration, and a section configured to calculate a liquid content ratio of whole blood, which calculates a liquid content ratio of whole blood from the detection value obtained from the device for detecting the hemocytes/plasma ratio, predetermined liquid content ratio of hemocytes and predetermined liquid content ratio of plasma, wherein the section for calculating plasma glucose calculates a plasma glucose concentration by multiplying a calculated value obtained from the section for calculating the whole blood glucose concentration with a multiplying factor of the liquid content ratio of plasma divided by a calculated value of the liquid content ratio of whole blood and outputs such resultant calculated value of the plasma glucose concentration.

The system for measuring plasma glucose of this invention allows for plasma glucose concentration measurement with accuracy using blood as a specimen.

This configuration allows for calculation of the hemocyte/plasma ratio in blood with accuracy and exact calculation of the plasma glucose concentration, and allows for measurement of the plasma glucose concentration with accuracy using blood as a specimen.

In the system for measuring plasma glucose of this invention, the value correlated with the hemocyte/plasma ratio of the blood is preferably a hemoglobin concentration of the measurement sample.

The device for detecting whole blood glucose which configures these systems preferably comprises, for example, a glucose sensor. Also, the device for detecting the hemocytes/plasma ratio preferably comprises, for example, an absorption spectrometer.

Also, a blood analysis system (hereinafter referred to as "the blood analysis system of this invention") can be further constructed, which carries out an analysis required for a diagnosis of a disease, such as diabetes, by systemizing the devices for measuring plasma glucose of this invention, a glycohemoglobin measuring device for measuring a glycohemoglobin concentration in the blood, a diabetes diagnosing device for determining a possibility of diabetes based on measured values obtained from the system for measuring plasma glucose of this invention and the glycohemoglobin measuring device, a device required for a diagnosis of other illnesses as necessary, or the like.

This allows for a more exact diabetes diagnosis (including a follow-up).

In the blood analysis system of this invention, the device for measuring plasma glucose of this invention can also be replaced for the system for measuring plasma glucose of this invention.

The method for measuring plasma glucose of this invention can obtain a measured value extremely close to an actual plasma glucose concentration with a hemolyzed sample obtained by hemolyzing hemocytes in blood because a hemocyte/plasma ratio in blood, liquid content ratio of hemocytes and liquid content ratio of plasma is used in the plasma glucose concentration measurement. A balance can be maintained between analysis speed and measurement accuracy of a plasma glucose concentration because the method for measuring plasma glucose of this invention can easily calculate the plasma glucose concentration from a whole blood glucose concentration.

The device for measuring plasma glucose of this invention can output a calculated value extremely close to an actual plasma glucose concentration because it has each type of calculating sections for carrying out a characterized calculating which reflects a hemocyte/plasma ratio in blood, and liquid content ratio of hemocytes and liquid content ratio of plasma to obtain a calculated value of the plasma glucose concentration. A balance can also be maintained between analysis speed and measurement accuracy of the plasma glucose concentration because the device for measuring plasma glucose of this invention can easily calculate the plasma glucose concentration from a whole blood glucose concentration.

The system for measuring plasma glucose and the blood analysis system of this invention can also obtain an accurate plasma glucose concentration, similarly to the above device for measuring plasma glucose. In addition, by systemizing a publicly known device for detecting glucose, absorption spectrometer, or the like and the calculating device for executing a characteristic calculating in this invention as described above, a highly accurate plasma glucose concentration can be obtained with utilizing conventional facilities, similarly to the device for measuring plasma glucose of this invention as described above. It can also be carried out from a remote location to process a specimen, obtain each detection value from the measurement sample, and process the detection value.

Certain preferred embodiments of the present invention will now be described in greater detail by way of example only and with reference to the accompanying drawings, in which:
FIG. 1 is an exemplary diagram showing a configuration of a preferred measurement device related to this invention;
FIG. 2 is a flowchart of a preferred measurement control for a measurement sample, which is carried out in the measurement device shown in FIG. 1;
FIG. 3 is a diagram which explains a preferred hemolysis progress occurring in a diluted bath while the measurement control shown in FIG. 2 is being carried out; and
FIG. 4 is a schematic diagram of an internal configuration of a preferred measurement device (micro device) related to this invention.

The method for measuring plasma glucose of this invention is explained below.

It is preferred that a measurement sample used for the method for measuring plasma glucose of this invention should be prepared by preliminarily carrying out a sample preparation step when whole blood glucose is measured. In this sample preparation step, the measurement sample is prepared by hemolyzing hemocytes in blood.

In this invention, "blood" simply referred as to means withdrawn blood.

Also, in this invention, "whole blood" referred as to means all components included in blood.

The sample preparation step may include diluting blood. The sample preparation step may also include hemolyzing hemocytes. A method for hemolyzing hemocytes includes a method of using a hemolytic agent, ultrasound, or the like. Of these methods, the one of using the hemolytic agent is preferred in terms of its ease. In this case, the methods of preliminarily mixing diluent and a hemolytic agent used for diluting blood to mix and stir the diluent including the hemolytic agent and the blood, and mixing and stirring diluent, a hemolytic agent, and blood together are preferred. A preparation of the measurement sample can be speeded up because these methods can carry out blood dilution and hemocyte hemolysis together and at the same time facilitate the hemocyte hemolysis.

As the hemolytic agent, ones usually used can be used, which includes a surface active agent of a glycoside of saponin and the like, polyoxyethylene alkyl aryl ether, higher fatty alcohol, a sulfonate or sulfate compound of polyoxyethylene ether, a polyoxyethylene adduct of sorbit fatty acid ester.

In the method for measuring plasma glucose of this invention, the glucose concentration in whole blood is measured with the measurement sample in the step of measuring whole blood glucose.

This glucose concentration can be measured with a so-called enzyme electrode method of detecting, with an electrode, an oxidation-reduction current of a product caused by an enzyme reaction with glucose and a glucose oxidoreductase, a so-called enzymatic colorimetric method (colorimetric method) of colorimetrically quantitating a reaction product caused by reacting an enzyme reaction product of glucose with a particular reagent (enzyme or chromogen), or the like.

In the enzyme electrode method, a glucose oxidase (GOD) or glucose dehydrogenase (GDH) is used as the glucose oxidoreductase.

If the enzyme electrode method is used, it is preferred that a voltage application to the electrode should be started in the step of measuring whole blood glucose when or before blood is added to the diluent including a hemolytic agent in the sample preparation step.

Generally, in the enzyme electrode method, it takes predetermined time (about 1 or 2 seconds) from starting the voltage application to the electrode to obtaining a stable current value reflecting a concentration of a measurement object substance in the sample. Therefore, such time can be shortened by starting the voltage application to the electrode without waiting for preparation completion of the measurement sample.

The whole blood glucose concentration is calculated according to a calibration curve (map) preliminarily created with standard glucose solution (standard solution) of a predetermined concentration when the current value of reflecting the glucose concentration of the measurement sample is obtained.

In the enzymatic colorimetric method, a hexokinase (HX) method, a glucose oxidase/peroxidase (GOD/POD) method or the like can be used.

In method for measuring plasma glucose of this invention, a hemocyte/plasma ratio in the blood can be obtained with any methods publicly known by those skilled in the art. The method for measuring plasma glucose of this invention includes a step of calculating a hemocytes/plasma ratio, which calculates the hemocyte/plasma ratio in the blood with the measurement sample. In this step of calculating a hemocytes/plasma ratio, for example, a value of reflecting a hemoglobin concentration of the measurement sample is measured, with which a hemoglobin concentration in whole blood can be calculated according to the calibration curve (map) preliminarily created with a predetermined concentration of the standard hemoglobin solution (standard solution) to calculate a ratio of red hemocytes in the blood from this hemoglobin concentration. For example, the hemoglobin concentration can be measured by the absorptiometry in terms of ease and accuracy. When an absorbance is obtained, the hemoglobin concentration in whole blood can be calculated according to the calibration curve (map) preliminarily created with the predetermined concentration of the standard hemoglobin solution (standard solution). Subsequently the ratio of hemocytes in blood is calculated from the calculated hemoglobin concentration. The method for calculating a ratio of hemocytes in blood from a hemoglobin concentration is known in the art. In addition, as another method for calculating a ratio of hemocytes in blood, a method of centrifuging blood to obtain a ratio of separated hemocyte components is included. Because determining a ratio of hemocytes necessarily decides a ratio of plasma, a hemocyte/plasma ratio in blood can be calculated.

Furthermore, this step of calculating a hemocytes/plasma ratio is integrated with a following step of calculating a liquid content ratio of whole blood.

In the step of calculating a liquid content ratio of whole blood, the liquid content ratio of whole blood is measured from the hemocyte/plasma ratio in blood, and the predetermined values of liquid content ratios of hemocytes and of plasma. Here, a liquid content ratio of hemocytes means a volume ratio of liquid content among hemocytes, a liquid content ratio of plasma means a volume ratio of liquid content among plasma, and a liquid content ratio of whole blood means a volume ratio of liquid content among whole blood. Either of the liquid content ratios of hemocytes or plasma can be preliminarily obtained in an experiment, or a predetermined value which is preliminarily decided can be used as a constant in this step.

The liquid content ratio of whole blood can be obtained, for example, by adding a value obtained by multiplying the ratio of hemocytes in blood by the liquid content ratio of hemocytes to a value obtained by multiplying a ratio of plasma in blood by the liquid content ratio of plasma.

In the step of calculating plasma glucose, the plasma glucose concentration is calculated from the whole blood glucose concentration measured in the step of measuring whole blood glucose with the liquid content ratio of whole blood calculated in the step of calculating a liquid content ratio of whole blood. As described, the method for calculating the plasma glucose concentration includes calculating the plasma glucose concentration by multiplying the whole blood glucose concentration measured in the step of measuring whole blood glucose by a multiplying factor of the liquid content ratio of plasma divided by the liquid content ratio of whole blood.

A measured value obtained in the step of measuring whole blood glucose is a glucose concentration in all components of blood (whole blood) obtained from the measurement sample whose hemocytes in blood are hemolyzed. Therefore, according to such calculating, such measured value can be converted into the plasma glucose concentration originally intended to obtain. Here, the liquid content ratio of plasma is to be the same value as the liquid content ratio of plasma used in the above step of calculating a liquid content ratio of whole blood.

The foregoing step of calculating a hemocytes/plasma ratio, step of calculating a liquid content ratio of whole blood, and step of calculating plasma glucose are described with dividing into each step for simplicity to explain meanings of the calculating, and it goes without saying that part of these steps can be integrated or broken down as long as the equivalent calculating is finally carried out.

Preferably the method for measuring plasma glucose of this invention further includes a correction step for correcting an influence which an enzymatic reaction rate of an unlysed blood sample before finishing hemolyzing all hemocytes in the sample preparation step exerts an enzymatic reaction rate of the measurement sample.

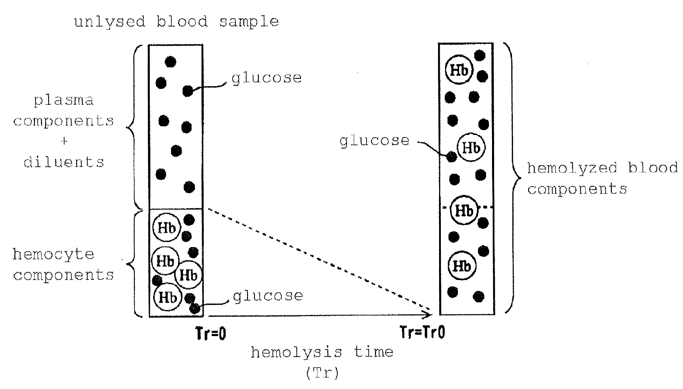

This correction step is explained with reference to FIG. 3.

FIG. 3 shows a change in a sample in the sample preparation step. The hemolysis time when Tr is 0 corresponds to a time immediately before or when an operation for hemolyzing hemocytes in blood (e.g. an addition of diluent including a hemolytic agent to blood) starts. A true dilution factor is larger than an apparent dilution factor to a specimen because the sample in this state is in a state where the diluent dilutes only liquid components present outside an unlysed hemocyte membrane.

When the hemolysis time Tr is passed, cell membranes of the hemocytes (mostly red hemocytes) are broken and components including glucose and hemoglobin (shown as Hb in the diagram) contained within them is diffused into a mixture of a plasma components and the diluent. Then, the hemolysis time Tr leads to a reference hemolysis time of Tr0 (when the hemocytes are completely hemolyzed).

Therefore a current gets generated correlating with the glucose concentration of the sample in which unlysed hemocytes are present (referred to as "unlysed blood sample" in this invention) until a time has passed (Tr = Tr0) to be taken to completely hemolyze hemocytes in blood since the operation for the hemolysis of hemocytes in blood began (Tr = 0).

Namely, the current gets generated correlating with a lower concentration than the glucose concentration of the measurement sample in the state where its hemocytes has been completely hemolyzed before the predetermined time passes.

Thus, as mentioned above, a phenomenon occurs that the current which has been generated until hemocytes in blood are completely hemolyzed is below the one (reference current) correlating with the glucose concentration of the measurement sample in the state where its hemocytes are completely hemolyzed, when the voltage application to the electrodes is tried to be started without waiting for the preparation completion of the measurement sample in terms of analysis speed.

These kinds of phenomena have an influence toward extending time till a stabilization of a current amount which reflects the glucose concentration of the measurement sample. In other words, an enzymatic reaction rate of the unlysed blood sample is relatively slower than one of the measurement sample relatively early after a voltage application to electrodes is started, which has an influence toward making a current slightly smaller at a predetermined detection point.

It is believed that this kind of phenomena similarly occurs in the measurement with the enzymatic colorimetric method.

Therefore, the plasma glucose concentration can be calculated with extremely high accuracy by correcting the influence which the enzymatic reaction rate of the unlysed blood sample before the hemolysis completion of the whole hemocytes in the sample preparation step exerts on the enzymatic reaction rate of the measurement sample even when current detection or measurement of dye amounts of the measurement sample is carried out early after the beginning of the sample preparation step (beginning of hemolysis operation).

These corrections can be carried out, for example, by performing a regression analysis using the calculated value, as an explanatory variable, of the plasma glucose concentration from plural hemolyzed samples with variable glucose concentrations and the measured value, as an objective variable, measured using plural plasma samples with variable glucose concentrations obtained from the method for measuring plasma glucose of this invention to obtain a coefficient of showing a correlation of the both values.

The following explains each configuration of a device for measuring plasma glucose of this invention.

The device for measuring plasma glucose of this invention may comprise a sample preparation mechanism for preparing the measurement sample whose hemocytes in blood are hemolyzed. This sample preparation mechanism has, for example, a sample preparation bath (referred as to "a diluted bath" in the example), and also has a configuration including a nozzle and pump for dispensing blood (specimen) from a blood collection tube into a sample preparation bath, diluent required for preparing a measurement sample, a nozzle, supply path, and pump for supplying a reagent, such as a hemolytic agent, from a reagent bath into the sample preparation bath, or the like. Naturally, for the device for measuring plasma glucose of this invention, a micro device may be used. In this case, the sample preparation mechanism includes, for example, a flow path for dilution, which is a capillary.

The device for measuring plasma glucose of this invention comprises a section for measuring whole blood glucose, which measures a glucose concentration of whole blood with a measurement sample prepared by the sample preparation mechanism. As this section for measuring whole blood glucose, devices which detect an oxidation-reduction current of an enzyme reaction product caused by an enzyme reaction of glucose and a glucose oxidoreductase to convert the detection result into a glucose concentration, and the ones which detect an amount of dye caused by reacting an enzyme reaction product of glucose with a particular reagent to convert the detection result into a glucose concentration, and the like can be used.

For example, if the section for measuring whole blood glucose is the former device, the section for measuring whole blood glucose has a sensor section, a power supply section, a current value measurement section, a section for calculating a whole blood glucose concentration, or the like.

The sensor section outputs an electric physical quantity (current value) depending on a quantity of giving or receiving electrons to glucose in the measurement sample. The sensor section includes an enzyme-immobilized layer for immobilizing a glucose oxidoreductase, and an electrode for detecting an enzymatic reaction rate of glucose and a glucose oxidoreductase. The power supply section applies a voltage to the electrode. The current value measurement section also measures a current occurring between the electrodes and outputs a measured value.

The section for calculating a whole blood glucose concentration also calculates the whole blood glucose concentration from a value correlated with the glucose concentration in the measurement sample whose hemocytes in blood are hemolyzed, that is a current value measured in the measurement section, according to a predetermined conversion formula (map).

The device for measuring plasma glucose of this invention preferably comprises a section for calculating a hemocytes/plasma ratio, which calculates a hemocyte/plasma ratio in blood.

The section for calculating a hemocytes/plasma ratio, for example, measures a hemoglobin concentration of whole blood with the measurement sample to calculate a hemocyte/plasma ratio in blood from the measured value.

In this case, the section for calculating a hemocytes/plasma ratio has an absorption spectrometer, a section for calculating a hemoglobin concentration, a section for calculating a hemocyte ratio, a plasma ratio calculating section, or the like.

The absorption spectrometer, for example, irradiates light with an absorption wavelength of oxyhemoglobin to measure its absorbance.

The absorption spectrometer has a photometric cell, a light source, a received ray system for measurement, or the like. The photometric cell is for specifying a photometric area. As the light source, a laser diode can be used. The light source emits the light of the absorption wavelength of hemoglobin to the measurement sample in the photometric cell (a supply path in a certain mode). As the received ray system for measurement, a photodiode can be used. The received ray system for measurement receives light which have penetrated the measurement sample.

The section for calculating a hemoglobin concentration converts an absorbance measured by the absorption spectrometer into a hemoglobin concentration in whole blood according to a predetermined conversion formula (map). The section for calculating a hemocyte ratio converts the hemoglobin concentration in whole blood calculated by the section for calculating a hemoglobin concentration into a ratio of hemocytes in blood according to the predetermined conversion formula (map). The plasma ratio calculating section calculates a plasma ratio in blood from the ratio of hemocytes in hemocytes measured by the section for calculating a hemocyte ratio. The calculating method is as explained in the method for measuring plasma glucose of this invention above.

The device for measuring plasma glucose of this invention comprises a section for calculating a liquid content ratio of whole blood, which calculates a liquid content ratio in whole blood from a hemocyte/plasma ratio in blood, liquid content ratio in hemocytes and liquid content ratio of in plasma.

The calculating flow is as explained on the method for measuring plasma glucose of this invention above.

The device for measuring plasma glucose of this invention comprises a section for calculating plasma glucose, which calculates a plasma glucose concentration by multiplying the measured value obtained from the section for measuring whole blood glucose by a multiplying factor of the liquid content ratio of plasma divided by the calculated value obtained from the section for calculating a liquid content ratio of whole blood to output such resultant calculated value of the plasma glucose concentration.

The calculating flow is as explained on the method for measuring plasma glucose of this invention above.

The classification of the sample preparation mechanism, the section for measuring whole blood glucose, the section for calculating a hemocytes/plasma ratio, the section for calculating a liquid content ratio of whole blood, and the section for calculating plasma glucose as mentioned above is for associating the device configuration with the method for measuring plasma glucose of this invention, and the sections which have calculating functions in these mechanism and sections can be naturally integrated into one calculating section. As the calculating sections, a computer is used which includes a CPU, memory, hard disk, or the like, and the calculating is realized by a program running on such computer. A measurement control section is electrically connected to the sensor section and the absorption spectrometer, and obtains measurement data from the sensor section and the absorption spectrometer to carry out a predetermined calculating based on a preliminarily programmed calculating program. Also, these calculating sections can be integrated into a measurement control section which controls a drive system including a pump and a valve in a device, and in this case, such measurement control section is electrically connected to the drive system and outputs control signals for controlling this drive system based on a preliminarily programmed control program.

The device for measuring plasma glucose of this invention preferably has a correction calculating section for carrying out a calculating to correct an influence given to the enzymatic reaction rate of the measurement sample by the enzymatic reaction rate of the unlysed blood sample before the hemolysis completion of whole hemocytes as mentioned above. It goes without saying that this correction calculating section can be also included in the above calculating sections or measurement control sections.

The device for measuring plasma glucose of this invention can be applied to various sizes of devices for measuring plasma glucose. For example, as exemplarily shown in an example mentioned below, it can be applied to macro-sized as well as micro-sized devices.

A program for measuring plasma glucose of this invention is for making a computer work as the section for calculating the whole blood glucose concentration, the section for calculating the liquid content ratio of whole blood, the section for calculating plasma glucose, and further the section for calculating a hemocytes/plasma ratio in the section for measuring whole blood glucose which the device for measuring plasma glucose comprises.

This invention also provides a thing that records this kind of programs in a computer readable recording medium. A recording medium of recording such program can determine whether it is suitable to carry with the above sample transfer control by reading and running this recording medium program on the computer.

The computer readable recording medium herein refers to a recording medium which can accumulate information, such as data and a program, with an electric, magnetic, optical, mechanical, or chemical action and read them from a computer. A recording medium removable from a computer in these ones includes, for example, a floppy ® disk, magnetic optical disk, CD-ROM, CD-R/W, DVD, blu-ray disc, DAT, 8 mm tape, memory card, or the like. As a recording medium fixed in a computer, there is also a hard disk, ROM (read only memory) or the like.

A system for measuring plasma glucose of this invention comprises a device for detecting whole blood glucose, which detects a value correlated with a glucose concentration of a measurement sample whose hemocytes in blood are hemolyzed. The device for detecting whole blood glucose can include the one for detecting, with an electrode, an oxidation-reduction current of an enzyme reaction product caused by an enzyme reaction of glucose and a glucose oxidoreductase, that is a glucose sensor, and the one for detecting an amount of dye caused by reacting an enzyme reaction product of glucose with a particular reagent.

The system for measuring plasma glucose of this invention comprises a device for detecting a hemocytes/plasma ratio for detecting a value correlated with a hemocyte/plasma ratio in the blood. The value correlated with the hemocyte/plasma ratio in the blood is preferably a hemoglobin concentration of the measurement sample. In this case, the device for detecting the hemocytes/plasma ratio includes an absorption spectrometer, measures an absorbance of hemoglobin, and detects a value correlated with the hemoglobin concentration of the measurement sample. The value correlated with the hemocyte/plasma ratio in the blood is a concept including a value correlated with a ratio of hemocytes in blood.

The system for measuring plasma glucose of this invention comprises a calculating device for calculating a plasma glucose concentration based on each detection value obtained from the device for detecting whole blood glucose and the device for detecting the hemocytes/plasma ratio.

Such calculating device has a section for calculating a whole blood glucose concentration, a section for calculating plasma glucose, and a section for calculating a liquid content ratio of whole blood.

The section for calculating a whole blood glucose concentration calculates a glucose concentration of whole blood based on the detection value obtained from the device for detecting whole blood glucose. The section for calculating a liquid content ratio of whole blood calculates, for example, the liquid content ratio of whole blood from the detection value obtained from the device for detecting the hemocytes/plasma ratio and the predetermined liquid content ratio of hemocytes and liquid content ratio of plasma. The section for calculating plasma glucose calculates the plasma glucose concentration by multiplying the calculated value obtained from the section for calculating a whole blood glucose concentration by a multiplying factor of the liquid content ratio of plasma divided by the calculated value obtained from the section for calculating the liquid content ratio of whole blood, and output such resultant calculated value of the plasma glucose concentration.

The calculating method is as explained on the method for measuring plasma glucose of this invention and the device for measuring plasma glucose above.

In a system for measuring plasma glucose of this invention, the calculating device is preferably network-connected with the device for detecting whole blood glucose and the device for detecting the hemocytes/plasma ratio. Such devices for detecting whole blood glucose and for detecting the hemocytes/plasma ratio have a function as a transmitter for transmitting a set of each detection value and patient's data to the calculating device. Also, the calculating device has a function as a receiver for receiving this set of data.

A blood analysis system of this invention comprises the device for measuring plasma glucose of this invention. In the blood analysis system of this invention, the device for measuring plasma glucose of this invention can be substituted for the system for measuring plasma glucose of this invention.

The blood analysis system of this invention comprises a glycohemoglobin measuring device for measuring a glycohemoglobin concentration in blood. This can estimate how much the plasma glucose concentration was 1 to 2 months ago and comprehensively carry out a necessary analysis for a diabetes diagnosis.

The blood analysis system of this invention comprises a diabetes determination device for determining a possibility of diabetes based on a measured value obtained from the device for measuring plasma glucose of this invention or the system for measuring plasma glucose of this invention and the glycohemoglobin measuring device. The diabetes determination device, for example, determines whether each of the measured values exceeds a predetermined reference value (e.g. a border value which can determine normal) which had been preliminarily stored to output its result.

The blood analysis system of this invention may further comprise a device or the like required for a diagnosis of other illnesses and carry out a necessary analysis for a diagnosis of other disease as necessary.

The followings explain the device for measuring plasma glucose related to the modes for carrying out the invention with reference to the drawings. The device of this mode is a blood analysis device into which the device for measuring plasma glucose and the glycohemoglobin measuring device are integrated. It is noted that the following configurations of the modes for carrying out are illustrative and this invention is not limited to these configurations of the modes for carrying out. The blood analysis system can be also configured with giving each function of this blood analysis device for a plurality of devices to form a network as necessary.

### [Example 1]

### <Summary of Blood Analysis Device>

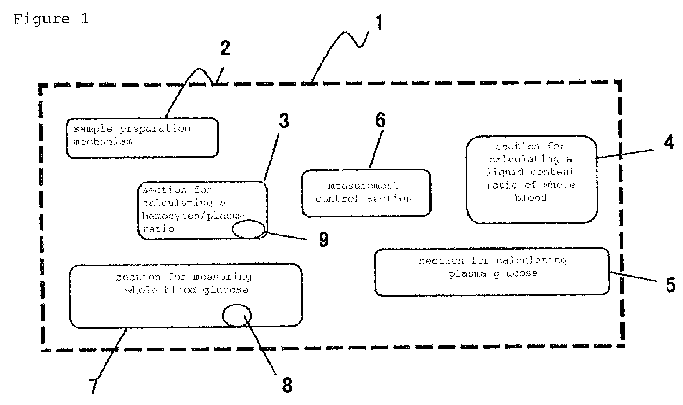

FIG. 1 is an exemplary diagram showing a configuration of a blood analysis device 1 related with this invention. The blood analysis device 1 is a device which automatically carries out a blood analysis about a diagnosis of diabetes in a person to be measured. In this example, by way of example, the blood analysis device 1 is explained, which measures plasma glucose and glycohemoglobin concentrations in blood to analyze a possibility of diabetes according to such measured values. The blood analysis device 1 has an aspiration nozzle, and the measurement is carried out through each process after blood externally carried with this nozzle is obtained in a blood collection tube.

In the blood analysis device 1, a predetermined configuration required for a blood analysis is placed inside. The device body configuring the blood analysis device 1 also comprises bottles which respectively accommodate liquid for analysis used for each kind of measurement, such as plasma glucose concentration measurement or glycohemoglobin concentration measurement. Specifically, it comprises, for example, a diluent bottle for accommodating diluent of diluting blood to be analyzed, a hemolytic bottle for accommodating hemolyzed blood including a hemolytic agent for hemolyzing hemocytes in blood, an elute bottle for accommodating elute used for a liquid chromatography for glycohemoglobin concentration measurement, or the like.

The hemolytic agent included by hemolyzed blood in the hemolytic bottle is for destructing cell membranes of hemocytes in blood. As the hemolytic agent, publicly known hemolytic agents can be employed, which are, for example, surface active agents, such as polyoxy (10) ethylene octyl phenyl ether (TritonX-10), higher fatty acid alcohol, alkyl aryl polyether alcohol, sulfonates of polyoxyethylene glycol, sulfates of polyoxyethylene ether, and polyoxyethylene derivatives of anhydrous sorbitol fatty acid ester, and publicly known hemolytic agents of ammonium chloride.

The hemolyzed blood can be used for measurement sample preparations with mixing with the diluent.

Here, a housing configuring the device body of the blood analysis device 1 provides an operation panel and a display panel (neither shown) on its surface section. On the operation panel, operation buttons are placed for operating the blood analysis device 1. When these operation buttons are operated by users of the device, a predetermined analytical processing for an analysis of blood be analyzed will be carried out. Also, the display panel shows results of the blood analysis carried out with the blood analysis device 1, operation procedures of the device, and a variety of information on diabetes diagnosis.

As described, the blood analysis device 1 obtains blood from the blood collection tube in response to an analysis order from a user, and adds diluent and hemolyzed blood to the obtained blood to prepare the measurement sample. Then, the prepared measurement sample will be provided for plasma glucose and glycohemoglobin concentration measurement. In this way, within one blood analysis device 1, two different analytical processes are carried out for blood of one specimen. Therefore, a summary of the configuration of the device body which allows for the analytical processes is explained based on FIG. 1.

FIG. 1 is an exemplary diagram showing the configuration of the blood analysis device 1 as mentioned above. In FIG. 1, for example, the descriptions of detailed configurations, such as a mechanism for carrying a blood collection tube, a dispensing mechanism for dispensing a specimen from a blood collection tube, and a drain mechanism for emitting a used sample, are omitted.

As illustrated, the blood analysis device 1 has a sample preparation mechanism 2, a section for calculating a hemocytes/plasma ratio 3, a section for calculating a liquid content ratio of whole blood 4, a section for calculating plasma glucose 5, a measurement control section 6, and a section for measuring whole blood glucose 7. The sample prepared from blood to be analyzed in the sample preparation mechanism 2 is sent to the section for measuring whole blood glucose 7, and a glucose concentration in whole blood (whole blood glucose concentration value) is measured there. It is noted that an illustrative configuration for glycohemoglobin concentration measurement is omitted in this figure. That is, the blood analysis device 1 can be also configured to send such sample to a glycohemoglobin measurement mechanism (not shown) separately installed in the device to carry out a liquid chromatography for the glycohemoglobin concentration there.

These sample preparation mechanism 2, section for calculating a hemocytes/plasma ratio 3, section for calculating a liquid content ratio of whole blood 4, section for calculating plasma glucose 5, section for measuring whole blood glucose 7, and glycohemoglobin measurement mechanism (not shown) are electrically connected to the measurement control section, and transmit each kind of data to such measurement control section and carry out predetermined movements and processing according to action instructions from such measurement control section. This measurement control section corresponds to a computer, and the measurement control of glycohemoglobin and plasma glucose concentrations at the blood analysis device 1 are realized by a computer program which is run on such computer including an unshown CPU, memory, hard disk, or the like. The aforementioned operation and display panels are also connected to this measurement control section, and program processing related to operation instructions from users and indication to users is performed there. The configuration of the blood analysis device 1 is explained below.

The sample preparation mechanism 2 includes a diluted bath (not shown) where the specimen (blood) to be analyzed is mixed with the diluent and hemolyzed blood to prepare the measurement sample. An action of a hemolytic agent in this hemolyzed blood causes hemolysis of hemocyte components, especially red hemocytes, in blood within the diluted bath to prepare the measurement sample with its hemocytes hemolyzed.

The measurement sample prepared at this diluted bath is now provided for an analytical processing at the section for measuring whole blood glucose 7. The section for measuring whole blood glucose 7 has a sensor section 8, a power supply section (not shown), and a current value measurement section (not shown). The sensor section 8 is for outputting an electric physical quantity (current value) in response to an amount of accepting electrons from and receiving electrons to glucose in the measurement sample, and has electrodes of accepting electrons from and receiving electrons to an enzyme-immobilized layer with its glucose oxidoreductase immobilized and an enzyme reaction product on the enzyme-immobilized layer. In addition, as the oxidoreductase included in this enzyme-immobilized layer, a glucose oxidase (GOD) and a glucose dehydrogenase (GDH) can be employed, and if the GOD is employed as such enzyme, hydrogen peroxide electrodes will be employed as the corresponding electrodes. This example below is explained on the assumption that the GOD is employed as such enzyme, but this is not intended to limit possibly employed enzymes to the GOD.

It is noted that the glucose measurement can be also carried out with a colorimetric method other than the enzyme electrode method, as mentioned above.

Current flows between electrodes in the sensor section 8 when the power supply section applies voltage with the sensor section exposed to the measurement sample. The current value measurement section measures the current between these electrodes to send its measured value to the measurement control section.

The section for calculating a hemocytes/plasma ratio 3 has an analysis area (not shown) which has an absorption spectrometer 9. The absorption spectrometer 9 irradiates an absorption wavelength of oxyhemoglobin to the measurement sample set in the analysis area to measure its absorbance. The absorption spectrometer 9 has a laser diode for emitting and a photodiode for receiving. The laser diode for emitting emits to the measurement sample which flows in the analysis area and the photodiode for receiving receives light which penetrates such measurement sample.

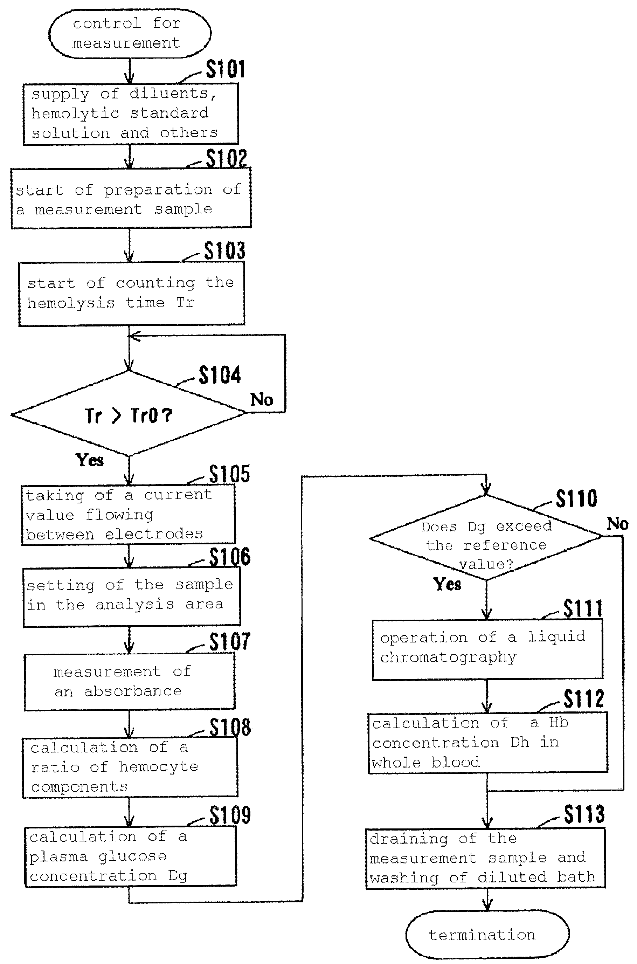

The measurement control of plasma glucose and glycohemoglobin concentrations for diabetes diagnosis in the blood analysis device 1 is explained based on FIG. 2. The measurement control shown in FIG. 2 is a measurement processing of the foregoing indicators for diabetes diagnosis realized by running a program recorded in a memory in the measurement control section 6 which is also a computer. Such measurement control is specifically performed when a user operates the operation panel (not shown) on the blood analysis device 1 to give an instruction of starting measurement.

First, at S101, preparation substances (diluent except blood, hemolyzed blood, and others) for preparing the measurement sample are provided in the diluted bath in the sample preparation mechanism 2. When the processing of S101 is finished, it proceeds to S102.

At S102, blood (whole blood) to be analyzed is added to the diluted bath provided with the preparation substances to prepare the measurement sample. When the processing of S102 is finished, it proceeds to S 103.

In S103, counting hemolysis time Tr is started. The hemolysis time Tr is an elapsed time when blood included in the measurement sample is subjected to the action of the hemolytic agent in hemolytic standard solution, and, in this example, a timing of its start (Tr = 0) is a time of starting processing of S102. Also, at the same time that counting the hemolysis time Tr is started, a stirrer located in the diluted bath starts agitation. This agitation will promote hemolysis. When the processing of S103 is finished, it proceeds to S104.

At S104, it is determined whether the hemolysis time Tr exceeds a reference hemolysis time Tr0. This reference hemolysis time Tr0 is decided considering a balance between a hemolysis progress in blood and an analysis time in the blood analysis device 1, especially the measurement time of a glucose concentration. For example, the hemolysis time which can maintain balance between the hemolysis progress and a processing capacity of the blood analysis device can be set as the reference hemolysis time Tr0. When it is determined affirmative at S104, it proceeds to S105, and when it is determined negative, the processing of S104 is repeated.

At S105, a detection value, that is a current value flowing between the hydrogen peroxide electrodes, is obtained by the sensor section 8 of the section for measuring whole blood glucose 7. In this example, at the time when the hemolysis time Tr reaches the reference hemolysis time Tr0, such current value flowing between the electrodes is measured by the current value measurement section and its measured value passes to the measurement control section 6.

When the processing of above S105 is finished, it proceeds to S 106. At S106, at least part of the measurement sample in the diluted bath is set in the analysis area of the hemocytes/plasma ratio section so that the hemoglobin concentration based on its absorbance can be measured by the absorption spectrometer. When the processing of S106 is finished, it proceeds to S107.

At S107, an absorbance of the absorption wavelength of hemoglobin of the measurement sample is measured by the absorption spectrometer 9, and its measured value passes to the measurement control section 6. In the measurement control section 6, a hemoglobin concentration in the measurement sample is calculated based on its measured value. When the processing of S 107 is finished, it proceeds to S 108.

At S108, based on the hemoglobin concentration calculated at S107, a ratio of hemocyte components of the measurement sample is calculated. This ratio of hemocyte components corresponds to a percentage for which the hemocyte components accounts in whole blood, as shown on the left side of FIG. 3. That is, in this example, at S108, the percentage for which the hemocyte components accounts in whole blood before hemolysis is derived from the hemoglobin concentration of liquid components of blood in hemolyzed state shown on the right side of FIG. 3. Considering that there is a correlation between such hemoglobin concentration and the ratio of hemocyte components, in this example, a mode is employed, where the ratio of hemocyte components is derived from the absorbance directly obtained by the absorption spectrometer 9. When the processing of S108 is finished, it proceeds to S109.

At S109, a plasma glucose concentration Dg to be analyzed is calculated based on an interelectrode current value obtained at S 105 and the ratio of hemocyte components calculated at S108.

Here, the whole blood glucose concentration calculated based on the interelectrode current value obtained at S105 is a glucose concentration in the measurement sample with its hemocytes hemolyzed, namely whole blood calculated based on the interelectrode current value occurring in the state shown on the right side of FIG. 3. In this state, it is the glucose concentration in the measurement sample where liquid components included in hemocytes and plasma are summed. Therefore, to convert such glucose concentration into the plasma glucose concentration Dg required for diabetes diagnosis, in this example, the above glucose concentration in whole blood (whole blood glucose concentration value) is multiplied by a multiplying factor, to a liquid content ratio of plasma, of a liquid content ratio of whole blood at the time of measuring the whole blood glucose concentration value. This can calculate a glucose value extremely close to an actual plasma glucose value.

In this step, the plasma glucose concentration Dg may be corrected, considering a gap between the reference current and the measurement current caused by an elapsed time of hemolysis. As mentioned above, a current value of an unlysed blood sample whose hemocytes has not completely been hemolyzed may have been detected until the hemolysis time Tr reaches the reference hemolysis time Tr0. That is, an enzymatic reaction rate of the unlysed blood sample is relatively slower than one of the measurement sample relatively early after a voltage application to electrodes is started, which has an influence toward making a current slightly smaller at a predetermined detection point.

The influence which the enzymatic reaction rate of the unlyzed blood sample before the hemolysis completion of whole hemocytes in this sample preparation step has on the enzymatic reaction rate of the measurement sample appears as a gap between the above both currents. Then, it is contemplated that, as a result, this gap would have some influence on the ratio of hemocyte components calculated at S 108. Thus, in this example, a further correction coefficient may be added to suppress the influence of this gap.

This correction coefficient can be set based on the gap between the reference and measurement currents obtained in a pre-experiment at the time when the hemolysis time Tr reaches the reference hemolysis time Tr0. This correction can be carried out, for example, by a division or addition with the correction coefficient for the plasma glucose concentration Dg.

When the processing of S109 is finished, it proceeds to S110. At S110, it is determined whether the plasma glucose concentration Dg calculated at S 109 exceeds the reference value for diabetes diagnosis. If it is determined affirmative at S110, it means that a person to be measured has a possibility of diabetes on the basis of the glucose concentration in the diabetes diagnosis, and in this case, the processing of and after S111 is carried out. On the other hand, if it is determined negative at S110, it means that such person to be measured has a low possibility of diabetes, so in this case, a measurement of a glycohemoglobin concentration is avoided and it proceeds to S113. This can keep a person to be measured with a low possibility of diabetes from obtaining surplus measurement.

At S111, as mentioned above, the glycohemoglobin measurement mechanism 7 carries out a liquid chromatography, and then the glycohemoglobin concentration Dh in blood is calculated (the processing of S 112). The processes carried out at S111 and S 112 have been already known, so the detailed explanations are omitted.

Finally, at S113, the measurement sample is drained from the diluted bath, and this measurement control is finished.

In this way, this measurement control calculates the ratio of hemocyte components of the blood to be analyzed from the absorbance detected by the absorption spectrometer 9. This absorbance is the one which reflects a components state of blood in the actual measurement sample for directly measuring and operating the measurement sample, or for emitting light and receiving transmitted light for the absorbance measurement. Therefore, it is believed that the ratio of hemocyte components calculated from such absorbance reflects the ratio of hemocyte components of the actual measurement sample well, which allows for higher accurate measurement than plasma glucose concentration measurement techniques with prior technologies.

### <Variation>

The plasma glucose concentration measurement of utilizing the absorbance of the measurement sample as mentioned above can apply to not only micro-sized blood analysis devices with the aforementioned diluted bath, nozzle, or the like, but also any forms of blood analysis devices, for example, the ones using micro devices. In the micro devices, it allows for moving liquid, such as a sample, with utilizing a capillary phenomenon to form width and depth dimensions of a flow path of flowing samples and others according to an order from a micrometer. Utilizing these micro devices can make the blood analysis devices small.

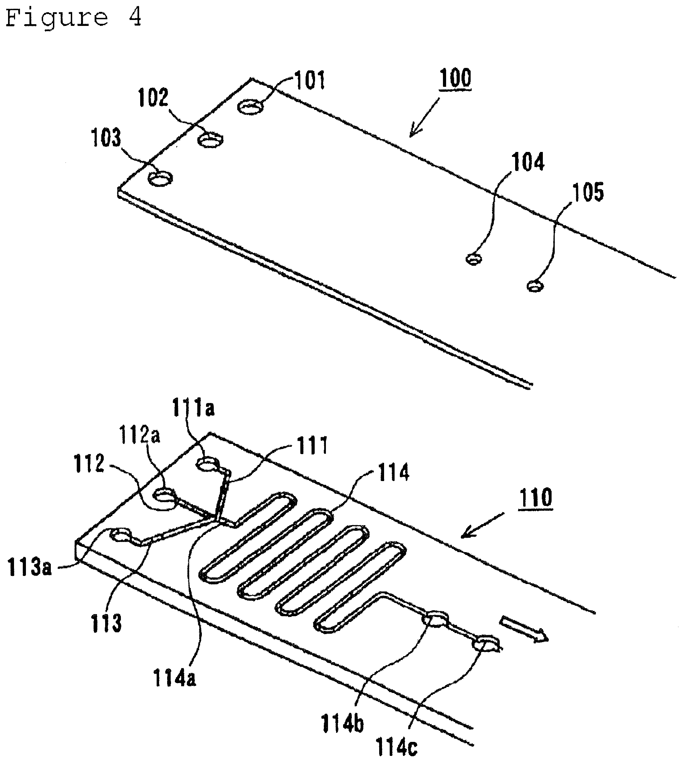

The micro device shown in FIG. 4 is the one which is configured to realize the same measurement as the plasma glucose concentration measurement shown in the above example. Specifically, the measurement of the whole blood glucose concentration by the electrodes with the GOD layers (corresponding to the measurement in the above sensor section 8), and the optical system measurement for measuring the absorbance (corresponding to the measurement in above 9) are carried out for the measurement sample prepared by mixing the blood to be analyzed and the diluent and the hemolysis standard solution, and finally the measurement of the plasma glucose concentration is realized. It is noted that a flow path configuration for the preparation of the measurement sample and the measurement of the plasma glucose concentration is mainly described in the micro device shown in FIG. 4 and the descriptions, such as the measurement device itself and the measurement control section, are omitted.

The micro device shown in FIG. 4 is formed by laminating a cover 100 on unshown junction sheet on a substrate 110 where the flow path is formed. On the substrate 110, the flow path having a concave cross-section is formed. The flow path provided on the substrate 110 has a first supply flow path 111, a second supply flow path 112, a blood supply flow path 113, and a dilution flow path 114. Because each of the first supply flow path 111, the second supply flow path 112, and the blood supply flow path 113 is connected to an end 114a of the dilution flow path 114 and such dilution flow path 114 has a winding shape like an accordion, equal mixing of blood, diluent, and hemolysis standard solution is realized. This allows the measurement sample included in its blood hemolyzed state to send by a capillary phenomenon to a whole blood glucose concentration measurement section 114b and a hemoglobin absorbance measurement section 114c located at a site beyond an accordion part of the dilution flow path 114. In this whole blood glucose concentration measurement section 114b, electrodes of a sensor with a GOD layer corresponding to the above sensor section 8 are placed, wherein the glucose concentration in the measurement sample is detected as a current value between the sensor electrodes. Also, there is a configuration that an air vent port 104 on the above cover 100 is correspondingly placed at a position of the whole blood glucose concentration measurement section 114b when the micro device is formed. In addition, in the hemoglobin absorbance measurement section 114c, an optical system measurement device corresponding to the above absorption spectrometer 85 is placed, wherein the absorbance of the measurement sample is measured. Also, there is a configuration that an air vent port 105 on the above cover 100 is correspondingly placed at a position of the hemoglobin absorbance measurement section 114c when the micro device is formed.

In the micro device configured in this way, the diluent is introduced from a first entrance 101, the hemolysis standard solution is introduced from a second entrance 102, and the blood is introduced from a blood entrance 103. A capillary phenomenon causes these kinds of liquid to go through the first supply flow path 111, the second supply flow path 112, and the blood supply flow path 113 respectively, to reach the dilution flow path 114, and to be mixed to form the measurement sample. While this measurement sample is proceeding in the dilution flow path, the blood is getting hemolyzed by the action of the hemolytic agent included in the hemolysis standard solution. Then, until the measurement sample reaches the whole blood glucose concentration measurement section 114b, most of the hemocyte components in the blood have been hemolyzed. There in the whole blood glucose concentration measurement section 114b, the whole blood glucose concentration in the measurement sample is measured, and further in the hemoglobin absorbance measurement section 114c, the absorbance of such measurement sample is measured. Each measurement processing corresponds to S105 and S 107 in the foregoing measurement control, and the calculation of the plasma glucose concentrations in blood based on these measurement results is as shown in the explanation on the measurement control in FIG. 2.

As described, as long as the above devices for realizing the method of measuring the plasma glucose concentration based on the glucose concentration in whole blood obtained with the hemolyzed measurement sample and the hemoglobin concentration in whole blood obtained from its hemoglobin absorbance comprise mechanisms of mixing blood, the hemolytic agent, and the diluent to prepare the measurement sample, of detecting the glucose concentration, and of detecting the hemoglobin concentration, and a computer for calculating aforementioned specific calculating, any of concrete shapes and dimensions of such mechanisms can be employed.

### [Explanation of the Symbols]

1····blood analysis device
2····sample preparation mechanism
3····section for calculating a hemocytes/plasma ratio
4····section for calculating a liquid content ratio of whole blood
5····section for calculating plasma glucose
6····measurement control section
7····section for measuring whole blood glucose
8····sensor section
9····absorption spectrometer
110····substrate
103····blood entrance
113····blood supply flow path
114····dilution flow path
114b····whole blood glucose concentration measurement section
114c····hemoglobin absorbance measurement section

## Claims

1. A method for measuring plasma glucose comprising calculating a plasma glucose concentration from a whole blood glucose concentration measured from a measurement sample prepared by hemolyzing hemocytes in blood, wherein the calculation is based on a hemocyte/plasma ratio in the blood, liquid content ratio of hemocytes and liquid content ratio of plasma in the blood,
the method comprising:
a step of measuring the glucose concentration in whole blood with a measurement sample prepared by hemolyzing hemocytes in blood,
a step of calculating a liquid content ratio of whole blood based on the hemocyte/plasma ratio in the blood, the liquid content ratio of hemocytes and the liquid content ratio of plasma, and
a step of calculating plasma glucose concentration from the glucose concentration in whole blood, based on the calculated liquid content ratio of whole blood, by multiplying the whole blood glucose concentration with a multiplying factor of the liquid content ratio of plasma divided by the liquid content ratio of whole blood.

2. The method of claim 1, further comprising a step of measuring a hemoglobin concentration in whole blood with the measurement sample to calculate the hemocyte/plasma ratio of blood.

3. The method of claim 2, wherein the hemoglobin concentration is measured by absorptiometry.

4. The method of any one of claims 1 to 3, further comprising a sample preparation step of adding and stirring blood in diluent containing a hemolytic agent.

5. The method of any one of claims 1 to 4, wherein the whole blood glucose concentration is measured by detecting an oxidation-reduction current of an enzyme reaction product of glucose and a glucose oxidoreductase using an electrode, or wherein the whole blood glucose concentration is measured by colorimetrically quantitating a reaction product of an enzyme reaction product of glucose and a chromogen.

6. The method of claim 5, further comprising a correction step of correcting an influence which an enzymatic reaction rate of an unlysed blood sample before finishing hemolysis of all hemocytes in the sample preparation step exerts on an enzymatic reaction rate of the measurement sample.

7. A device for measuring plasma glucose, comprising:
a section configured to measure whole blood glucose, which measures a glucose concentration in whole blood from a measurement sample prepared by hemolyzing hemocytes in blood,
a section configured to calculate plasma glucose, which calculates a plasma glucose concentration from the glucose concentration in whole blood based on a hemocyte/plasma ratio in the blood, the liquid content ratio of hemocytes and the liquid content ratio of plasma, and
a section configured to calculate a liquid content ratio of whole blood, which calculates a liquid content ratio of whole blood from the hemocyte/plasma ratio in the blood, predetermined liquid content ratio in hemocytes and predetermined liquid content ratio of plasma,
wherein the section for calculating plasma glucose calculates a plasma glucose concentration by multiplying a measured value of whole blood glucose concentration with a multiplying factor of the liquid content ratio of plasma divided by the calculated value of the liquid content ratio of whole blood and outputs such calculated value of the plasma glucose concentration.

8. The device of claim 7, further comprising a section for calculating a hemocyte/plasma ratio, which measures a whole blood hemoglobin concentration of the measurement sample and calculates a hemocyte/plasma ratio in blood from the hemoglobin concentration, and preferably wherein the section for calculating the hemocyte/plasma ratio has an absorption spectrometer for measuring the hemoglobin concentration.

9. The device of claim 7 or 8, comprising a sample preparation means for preparing the measurement sample in which hemocytes in blood are hemolyzed.

10. The device any one of claims 7 to 9, wherein the section for measuring whole blood glucose concentration has a sensor means including an enzyme-immobilized layer with immobilized glucose oxidoreductase and an electrode for detecting an oxidation-reduction current of an enzyme reaction product of glucose and a glucose oxidoreductase.

11. A program for measuring plasma glucose concentration to make a computer work as:
a section for calculating a whole blood glucose concentration, which calculates a glucose concentration in whole blood based on a value correlated with a glucose concentration of a measurement sample whose hemocytes in blood are hemolyzed,
a section for calculating plasma glucose, which calculates a plasma glucose concentration from a measured value of blood glucose concentration based on a hemocyte/plasma ratio in the blood, predetermined liquid content ratio of hemocytes and predetermined liquid content ratio of plasma and outputs such calculated value of the plasma glucose concentration, and
a section for calculating a liquid content ratio of whole blood, which calculates a liquid content ratio of whole blood from the hemocyte/plasma ratio in the blood, the predetermined liquid content ratio of hemocytes and the predetermined liquid content ratio of plasma,
wherein the section for calculating plasma glucose further calculates a plasma glucose concentration by multiplying a measured value of whole blood glucose concentration with a multiplying factor of the liquid content ratio of plasma divided by a calculated value of the liquid content ratio of whole blood and outputs such resultant calculated value of the plasma glucose concentration.

12. A program as claimed in claim 11, the program further being configured to make a computer work as a section for calculating a hemocyte/plasma ratio, which calculates a hemoglobin concentration in whole blood based on a value correlated with a hemoglobin concentration of the measurement sample and calculating a hemocyte/plasma ratio of blood from the hemoglobin concentration.

13. A computer-readable recording medium comprising the program of claim 11 or 12.

14. A system for measuring plasma glucose, comprising:
a device configured to detect whole blood glucose concentration, which detects a value correlated with a glucose concentration of a measurement sample prepared by hemolyzing hemocytes in blood,
a device configured to detect a hemocyte/plasma ratio, which detects a value correlated with a hemocyte/plasma ratio of the blood, and
a calculating device configured to calculate a plasma glucose concentration based on each detection value obtained from the devices for detecting whole blood glucose concentration and the hemocyte/plasma ratio, wherein the calculating device has
a section configured to calculate a whole blood glucose concentration, which calculates a glucose concentration in whole blood based on a detection value obtained from the device for detecting glucose concentration,
a section configured to calculate plasma glucose concentration, which calculates a plasma glucose concentration from the glucose concentration value in whole blood calculated at the section for calculating the whole blood glucose concentration, based on a detection value obtained from the device for detecting the hemocytes/plasma ratio, predetermined liquid content ratio in hemocytes and predetermined liquid content ratio of plasma and outputs such calculated value of the plasma glucose concentration, and
a section configured to calculate a liquid content ratio of whole blood, which calculates a liquid content ratio of whole blood from the detection value obtained from the device for detecting the hemocytes/plasma ratio, predetermined liquid content ratio of hemocytes and predetermined liquid content ratio of plasma,
wherein the section for calculating plasma glucose is configured to calculate a plasma glucose concentration by multiplying a calculated value obtained from the section for calculating the whole blood glucose concentration with a multiplying factor of the liquid content ratio of plasma divided by a calculated value of the liquid content ratio of whole blood and outputs such resultant calculated value of the plasma glucose concentration.

15. The system of claim 14, wherein the value correlated with the hemocyte/plasma ratio of the blood is a hemoglobin concentration of the measurement sample.

16. The system of claim 14 or 15, wherein the device for detecting whole blood glucose comprises a glucose sensor, and/or the device for detecting the hemocytes/plasma ratio comprises an absorption spectrometer.

17. The system of any one of claims 14, 15 or 16, further comprising:
a glycohemoglobin measuring device for measuring a glycohemoglobin concentration in the blood, and
a diabetes diagnosing device for determining a possibility of diabetes based on measured values obtained from the system for measuring plasma glucose and the glycohemoglobin measuring device.

## Patentansprüche

1. Ein Verfahren zum Messen von Plasma-Glucose aufweisend Berechnen einer Plasma-Glucose-Konzentration aus einer Vollblut-Glucose-Konzentration gemessen aus einer Messprobe hergestellt durch Hämolysieren von Hämozyten in Blut, wobei die Berechnung auf einem Hämozyt/Plasma-Verhältnis in dem Blut, einem Flüssigkeitsgehaltverhältnis von Hämozyten und einem Flüssigkeitsgehaltverhältnis von Plasma in dem Blut basiert, das Verfahren aufweisend:
einen Schritt von einem Messen der Glucose-Konzentration in Vollblut mit einer Messprobe hergestellt durch Hämolysieren von Hämozyten in Blut,
einen Schritt von Berechnen eines Flüssigkeitsgehaltverhältnisses von Vollblut basierend auf dem Hämozyt/Plasma-Verhältnis in dem Blut, dem Flüssigkeitsgehaltverhältnis von Hämozyten und dem Flüssigkeitsgehaltverhältnis von Plasma und
einen Schritt von Berechnen einer Plasma-Glucose-Konzentration aus der Glucose-Konzentration in Vollblut basierend auf dem berechneten Flüssigkeitsgehaltverhältnis von Vollblut durch Multiplizieren der Vollblut-Glucose-Konzentration mit einem Multiplikationsfaktor von dem Flüssigkeitsgehaltverhältnis von Plasma geteilt durch das Flüssigkeitsgehaltsverhältnis von Vollblut.

2. Das Verfahren nach Anspruch 1, weiter aufweisend einen Schritt von Messen einer Hämoglobin-Konzentration in Vollblut mit der Messprobe zum Berechnen des Hämozyt/Plasma-Verhältnisses von Blut.

3. Das Verfahren nach Anspruch 2, wobei die Hämoglobin-Konzentration durch Absorptiometrie gemessen wird.

4. Das Verfahren nach einem der Ansprüche 1 bis 3, weiter aufweisend einen Probenherstellungsschritt von Hinzufügen und Rühren von Blut in einem Verdünnungsmittel, das ein hämolytisches Mittel aufweist.

5. Das Verfahren nach einem der Ansprüche 1 bis 4, wobei die Vollblut-Glucose-Konzentration gemessen wird durch Detektieren eines Oxidations-Reduktions-Stroms von einem Enzym-Reaktionsprodukt von Glucose und einer Glucose-Oxidoreduktase unter Verwendung einer Elektrode oder wobei die Vollblut-Glucose-Konzentration gemessen wird durch farbmetrisch Quantifizieren eines Reaktionsprodukts von einem Enzym-Reaktionsprodukt von Glucose und eines Chromogens.

6. Das Verfahren nach Anspruch 5, weiter aufweisend einen Korrekturschritt von Korrigieren eines Einflusses, den eine enzymatische Reaktionsrate von einer nicht lysierten Blutprobe vor einem Beenden einer Hämolyse von allen Hämozyten in dem Probenherstellungsschritt auf eine enzymatische Reaktionsrate von der Messprobe ausübt.

7. Eine Vorrichtung zum Messen von Plasma-Glucose, aufweisend:
einen Abschnitt, der dazu konfiguriert ist, um Vollblut-Glucose zu messen, der eine Glucose-Konzentration in Vollblut aus einer Messprobe hergestellt durch Hämolysieren von Hämozyten in Blut misst,
einen Abschnitt, der dazu konfiguriert ist, um Plasma-Glucose zu berechnen, der eine Plasma-Glucose-Konzentration aus der Glucose-Konzentration in Vollblut berechnet, basierend auf einem Hämozyt/Plasma-Verhältnis in dem Blut, dem Flüssigkeitsgehaltverhältnis von Hämozyten und dem Flüssigkeitsgehaltverhältnis von Plasma, und
einen Abschnitt, der dazu konfiguriert ist, um ein Flüssigkeitsgehaltverhältnis von Vollblut zu berechnen, der ein Flüssigkeitsgehaltverhältnis von Vollblut aus dem Hämozyt/Plasma-Verhältnis in dem Blut, einem vorbestimmten Flüssigkeitsgehaltverhältnis in Hämozyten und einem vorbestimmten Flüssigkeitsgehaltverhältnis von Plasma berechnet,
wobei der Abschnitt zum Berechnen von Plasma-Glucose eine Plasma-Glucose-Konzentration durch Multiplizieren eines gemessenen Wertes von einer Vollblut-Glucose-Konzentration mit einem Multiplikationsfaktor von dem Flüssigkeitsgehaltverhältnis von Plasma geteilt durch den berechneten Wert von dem Flüssigkeitsgehaltverhältnis von Vollblut berechnet und einen solchen berechneten Wert von der Plasma-Glucose-Konzentration ausgibt.

8. Die Vorrichtung nach Anspruch 7, weiter aufweisend einen Abschnitt zum Berechnen eines Hämozyt/Plasma-Verhältnisses, welcher eine Vollblut-Hämoglobin-Konzentration von der Messprobe misst und ein Hämozyt/Plasma-Verhältnis in Blut aus der Hämoglobin-Konzentration berechnet, und wobei der Abschnitt zum Berechnen des Hämozyt/Plasma-Verhältnisses vorzugsweise ein Absorptions-Spektrometer zu Messen der Hämoglobin-Konzentration hat.

9. Die Vorrichtung nach Anspruch 7 oder 8, aufweisend ein Probenherstellungsmittel zum Herstellen des Messprobe in der Hämozyten in Blut hämolysiert werden.

10. Die Vorrichtung nach einem der Ansprüche 7 bis 9, wobei der Abschnitt zum Messen einer Vollblut-Glucose-Konzentration ein Sensormittel hat, das eine Enzym-immobilisierte Schicht mit immobilisierter Glucose-Oxidoreduktase und eine Elektrode zum Detektieren eines Oxidations-Reduktions-Stroms von einem Enzym-Reaktionsprodukt von Glucose und einer Glucose-Oxidoreduktase umfasst.

11. Ein Programm zum Messen von einer Plasma-Glucose-Konzen-tration, um einen Computer arbeiten zu lassen als:
einen Abschnitt zum Berechnen einer Vollblut-Glucose-Konzentration, welcher eine Glucose-Konzentration in Vollblut basierend auf einem Wert berechnet, der mit einer Glucose-Konzentration von einer Messprobe korreliert ist, deren Hämozyten hämolysiert sind,
einen Abschnitt zum Berechnen von Plasma-Glucose, welcher eine Plasma-Glucose-Konzentration aus einem gemessenen Wert von einer Blut-Glucose-Konzentration basierend auf einem Hämozyt/Plasma-Verhältnis in dem Blut, einem vorbestimmten Flüssigkeitsgehaltverhältnis von Hämozyten und einem vorbestimmten Flüssigkeitsgehaltverhältnis von Plasma berechnet und einen solchen berechneten Wert von der Plasma-Glucose-Konzentration ausgibt, und
einen Abschnitt zum Berechnen eines Flüssigkeitsgehaltverhältnisses von Vollblut, welcher ein Flüssigkeitsgehaltverhältnis von Vollblut aus dem Hämozyt/Plasma-Verhältnis in dem Blut, dem vorbestimmten Flüssigkeitsgehaltverhältnis von Hämozyten und dem vorbestimmten Flüssigkeitsgehaltverhältnis von Plasma berechnet,
wobei der Abschnitt zum Berechnen einer Plasma-Glucose weiter eine Plasma-Glucose-Konzentration durch Multiplizieren eines gemessenen Wertes von einer Vollblut-Glucose-Konzentration mit einem Multiplikationsfaktor von dem Flüssigkeitsgehaltverhältnis von Plasma geteilt durch einen berechneten Wert von dem Flüssigkeitsgehaltverhältnis von Vollblut berechnet und einen solchen resultierenden berechneten Wert von der Plasma-Glucose-Konzentration ausgibt.

12. Ein Programm wie in Anspruch 11 beansprucht, wobei das Programm weiter dazu konfiguriert ist, um einen Computer arbeiten zu lassen als einen Abschnitt zum Berechnen eines Hämozyt/Plasma-Verhältnisses, welcher eine Hämoglobin-Konzentration in Vollblut basierend auf einem Wert, der mit einer Hämoglobin-Konzentration von der Messprobe korreliert ist, berechnet und aus der Hämoglobin-Konzentration ein Hämozyt/Plasma-Verhältnis von Blut berechnet.

13. Ein computerlesbares Aufzeichnungsmedium, das das Programm nach Anspruch 11 oder 12 aufweist.

14. Ein System zum Messen einer Plasma-Glucose, aufweisend:
eine Vorrichtung, die dazu konfiguriert ist, um eine Vollblut-Glucose-Konzentration zu detektieren, welche einen Wert detektiert, der mit einer Glucose-Konzentration von einer Messprobe korreliert ist, die durch Hämolysieren von Hämozyten in Blut hergestellt ist,
eine Vorrichtung, die dazu konfiguriert ist, um ein Hämozyt/Plasma-Verhältnis zu detektieren, welche einen Wert detektiert, der mit einem Hämozyt/Plasma-Verhältnis von dem Blut korreliert ist, und
eine Berechnungsvorrichtung, die dazu konfiguriert ist, um basierend auf jedem Detektionswert erhalten von den Vorrichtungen zum Detektieren von einer Vollblut-Glucose-Konzentration und des Hämozyt/Plasma-Verhältnisses eine Plasma-Glucose-Konzentration zu berechnen, wobei die Berechnungsvorrichtung hat
einen Abschnitt, der dazu konfiguriert ist, um eine Vollblut-Glucose-Konzentration zu berechnen, welcher eine Glucose-Konzentration in Vollblut basierend auf einem Detektionswert erhalten von der Vorrichtung zum Detektieren einer Glucose-Konzentration berechnet,
einen Abschnitt, der dazu konfiguriert ist, um eine Plasma-Glucose-Konzentration zu berechnen, welcher eine Plasma-Glucose-Konzentration aus dem Glucose-Konzentrationswert in Vollblut berechnet, der an dem Abschnitt zum Berechnen der Vollblut-Glucose-Konzentration berechnet wird, basierend auf einem Detektionswert erhalten von der Vorrichtung zum Detektieren des Hämozyt/Plasma-Verhältnisses, einem vorbestimmten Flüssigkeitsgehaltverhältnis in Hämozyten und einem vorbestimmten Flüssigkeitsgehaltverhältnis von Plasma und einen solchen berechneten Wert von der Plasma-Glucose-Konzentration ausgibt, und
einen Abschnitt, der dazu konfiguriert ist, um ein Flüssigkeitsgehaltverhältnis von Vollblut zu berechnen, welcher ein Flüssigkeitsgehaltverhältnis von Vollblut aus dem Detektionswert erhalten von der Vorrichtung zum Detektieren des Hämozyt/Plasma-Verhältnisses, einem vorbestimmten Flüssigkeitsgehaltverhältnisses von Hämozyten und einem vorbestimmten Flüssigkeitsgehaltverhältnisses von Plasma berechnet,
wobei der Abschnitt zum Berechnen von Plasma-Glucose dazu konfiguriert ist, um eine Plasma-Glucose-Konzentration durch Multiplizieren eines berechneten Wertes erhalten von dem Abschnitt zum Berechnen der Vollblut-Glucose-Konzentration mit einem Multiplikationsfaktor von dem Flüssigkeitsgehaltverhältnis von Plasma geteilt durch einen berechneten Wert von dem Flüssigkeitsgehaltverhältnis von Vollblut zu berechnen und einen solchen resultierenden berechneten Wert von der Plasma-Glucose-Konzentration auszugeben.

15. Das System nach Anspruch 14, wobei der Wert, der mit dem Hämozyt/Plasma-Verhältnis von dem Blut korreliert ist, eine Hämoglobin-Konzentration von der Messprobe ist.

16. Das System nach Anspruch 14 oder 15, wobei die Vorrichtung zum Detektieren von Vollblut-Glucose einen Glucose-Sensor aufweist und/oder die Vorrichtung zum Detektieren des Hämozyt/Plasma-Verhältnisses ein Absorptions-Spektrometer aufweist.

17. Das System nach einem der Ansprüche 14, 15 oder 16, weiter aufweisend:
eine Glycohämoglobin-Messvorrichtung zum Messen einer Glycohämoglobin-Konzentration in dem Blut und
eine Diabetes-Diagnostizierungsvorrichtung zum Bestimmen einer Möglichkeit von Diabetes basierend auf gemessenen Werten erhalten von dem System zum Messen von Plasma-Glucose und der Glycohämoglobin-Messvorrichtung.

## Revendications

1. Procédé de mesure de glucose plasmatique comprenant le calcul d'une concentration de glucose plasmatique à partir d'une concentration de glucose sanguin mesurée en partant d'un échantillon de mesure préparé par hémolyse d'hémocytes du sang, dans lequel le calcul est basé sur un rapport hémocytes/plasma du sang, le rapport en teneur liquide des hémocytes et le rapport en teneur liquide du plasma dans le sang, le procédé comprenant :
une étape de mesure de la concentration de glucose dans du sang entier avec un échantillon de mesure préparé par hémolyse d'hémocytes dans le sang,
une étape de calcul d'un rapport en teneur liquide du sang entier sur la base du rapport hémocytes/plasma dans le sang, le rapport en teneur liquide d'hémocytes et le rapport en teneur liquide de plasma, et
une étape de calcul de la concentration de glucose plasmatique à partir de la concentration de glucose dans le sang entier, sur la base du rapport en teneur liquide calculé du sang entier, en multipliant la concentration de glucose du sang entier par un facteur de multiplication du rapport en teneur liquide du plasma divisé par le rapport en teneur liquide du sang entier.

2. Procédé selon la revendication 1, comprenant en outre une étape de mesure de la concentration d'hémoglobine dans le sang entier avec l'échantillon de mesure pour calculer le rapport hémocytes/plasma du sang.

3. Procédé selon la revendication 2, dans lequel la concentration d'hémoglobine est mesurée par absorptiométrie.

4. Procédé selon l'une quelconque des revendications 1 à 3, comprenant en outre une étape de préparation d'échantillon en ajoutant et en agitant du sang dans un diluant contenant un agent hémolytique.

5. Procédé selon l'une quelconque des revendications 1 à 4, dans lequel la concentration de glucose du sang entier est mesurée par détection d'un courant d'oxydoréduction d'un produit réactionnel enzymatique de glucose et oxydoréductase de glucose en utilisant une électrode, ou dans lequel la concentration de glucose du sang entier est mesurée par quantification colorimétrique d'un produit réactionnel d'un produit réactionnel enzymatique de glucose et d'un chromogène.

6. Procédé selon la revendication 5, comprenant en outre une étape de correction visant à corriger l'influence qu'un taux de réaction enzymatique d'un échantillon de sang non lysé avant finition de l'hémolyse de tous les hémocytes dans l'étape de préparation d'échantillon exerce sur un taux de réaction enzymatique de l'échantillon de mesure.

7. Dispositif de mesure de glucose plasmatique, comprenant :
une section configurée pour mesurer le glucose du sang entier, laquelle section mesure une concentration du glucose dans le sang entier à partir d'un échantillon de mesure préparé par hémolyse d'hémocytes dans le sang,
une section configurée pour calculer le glucose plasmatique, qui calcule une concentration en glucose plasmatique à partir de la concentration de glucose du sang entier basée sur un rapport hémocytes/plasma dans le sang, le rapport en teneur liquide des hémocytes et le rapport en teneur liquide du plasma et
une section configurée pour calculer un rapport en teneur liquide du sang entier, qui calcule un rapport en teneur liquide du sang entier à partir du rapport hémocytes/plasma dans le sang, du rapport en teneur liquide prédéterminé des hémocytes et un rapport en teneur liquide prédéterminé du plasma,
dans lequel la section de calcul du glucose plasmatique calcule une concentration de glucose plasmatique en multipliant la valeur mesurée de la concentration de glucose du sang entier avec un facteur de multiplication du rapport en teneur liquide du plasma divisée par la valeur calculée du rapport en teneur liquide du sang entier et fournit celle valeur calculée de la concentration de glucose plasmatique.

8. Dispositif selon la revendication 7, comprenant en outre une section pour calculer un rapport hémocytes/plasma qui mesure la concentration en hémoglobine du sang entier de l'échantillon de mesure et calcule un rapport hémocytes/plasma dans le sang à partir de la concentration d'hémoglobine et, de préférence, dans lequel la section de calcul du rapport hémocytes/plasma présente un spectromètre d'absorption pour mesurer la concentration d'hémoglobine.

9. Dispositif selon la revendication 7 ou 8, comprenant un moyen de préparation d'échantillon pour préparer l'échantillon de mesure dans lequel les hémocytes du sang sont hémolysées.

10. Dispositif selon l'une quelconque des revendications 7 à 9, dans lequel la section de mesure de la concentration de glucose du sang entier présente un moyen de détection comprenant une couche immobilisée par un enzyme avec une oxydoréductase de glucose immobilisée et une électrode pour détecter un courant d'oxydoréduction d'un produit enzymatique réactionnel de glucose et une oxydoréductase de glucose.

11. Programme de mesure de la concentration de glucose plasmatique pour élaborer un outil informatique comprenant :
une section de calcul d'une concentration de glucose du sang entier, qui calcule la concentration de glucose dans le sang entier sur la base d'une valeur corrélée avec une concentration de glucose d'un échantillon de mesure dont les hémocytes du sang sont hémolysés,
une section pour calculer le glucose plasmatique qui calcule la concentration de glucose plasmatique à partir d'une valeur mesurée de la concentration en glucose du sang sur la base d'un rapport hémocytes/plasma dans le sang, du rapport en teneur liquide prédéterminé des hémocytes et du rapport en teneur liquide prédéterminé du plasma et délivre cette valeur calculée de la concentration de glucose plasmatique et
une section pour calculer un rapport en teneur liquide du sang entier, qui calcule un rapport en teneur liquide du sang entier à partir du rapport hémocytes/plasma dans le sang, du rapport en teneur liquide prédéterminé des hémocytes et du rapport en teneur liquide prédéterminé du plasma,
dans lequel la section de calcul du glucose plasmatique calcule en outre une concentration de glucose plasmatique en multipliant une valeur mesurée de la concentration de glucose dans le sang entier par un facteur de multiplication du rapport en teneur liquide du plasma divisé par une valeur calculée du rapport en teneur liquide du sang entier et délivre cette valeur calculée obtenue de la concentration de glucose plasmatique.

12. Programme selon la revendication 11, le programme étant en outre configuré pour élaborer un outil de calcul tel qu'une section de calcul d'un rapport hémocytes/plasma, qui calcule une concentration d'hémoglobine dans le sang entier sur la base d'une valeur corrélée avec la concentration d'hémoglobine de l'échantillon de mesure et en calculant un rapport hémocytes/plasma du sang à partir de la concentration d'hémoglobine.

13. Support d'enregistrement lisible par ordinateur comprenant le programme de la revendication 11 ou 12.

14. Système de mesure de glucose plasmatique comprenant :
un dispositif configuré pour détecter la concentration de glucose du sang entier, qui détecte une valeur corrélée avec la concentration de glucose d'un échantillon de mesure préparé par hémolyse d'hémocytes dans le sang,
un dispositif configuré pour détecter un rapport hémocytes/plasma, qui détecte une valeur corrélée avec un rapport hémocytes/plasma du sang et
un dispositif de calcul configuré pour calculer une concentration de glucose plasmatique sur la base de chaque valeur de détection tirée des dispositifs de détection de la concentration de glucose du sang entier et le rapport hémocytes/plasma, dans lequel le dispositif de calcul présente :
une section configurée pour calculer une concentration de glucose du sang entier, qui calcule une concentration de glucose dans le sang entier sur la base d'une valeur de détection tirée du dispositif de détection de la concentration de glucose,
une section configurée pour calculer la concentration de glucose plasmatique qui calcule une concentration de glucose plasmatique à partir de la valeur de concentration de glucose dans le sang entier calculée dans la section de calcul de la concentration de glucose du sang entier sur la base d'une valeur de détection tirée du dispositif de détection du rapport hémocytes/-plasma, du rapport en teneur liquide prédéterminé des hémocytes et du rapport en teneur liquide prédéterminé du plasma et délivre cette valeur calculée de la concentration de glucose plasmatique et
une section configurée pour calculer un rapport en teneur liquide du sang entier, qui calcule un rapport en teneur liquide du sang entier à partir de la valeur de détection obtenue du dispositif de détection du rapport hémocytes/plasma, du rapport en teneur liquide prédéterminé des hémocytes et du rapport en teneur liquide prédéterminé du plasma,
dans lequel la section de calcul du glucose plasmatique est configurée pour calculer une concentration de glucose plasmatique en multipliant une valeur calculée obtenue de la section de calcul de la concentration de glucose dans le sang entier par un facteur de multiplication du rapport en teneur liquide du plasma divisée par une valeur calculée du rapport en teneur liquide du sang entier et délivre cette valeur calculée obtenue de la concentration de glucose plasmatique.

15. Système selon la revendication 14, dans lequel la valeur corrélée avec le rapport hémocytes/plasma du sang est une concentration d'hémoglobine de l'échantillon de mesure.

16. Système selon la revendication 14 ou 15, dans lequel le dispositif de détection du glucose dans le sang entier comprend un capteur de glucose et/ou le dispositif de détection du rapport hémocytes/plasma comprend un spectromètre d'absorption.

17. Système selon l'une quelconque des revendications 14, 15 ou 16, comprenant en outre :
un dispositif de mesure de la glycohémoglobine pour mesurer la concentration en glycohémoglobine dans le sang et
un dispositif de diagnostic du diabète pour déterminer une possibilité de diabète sur la base de valeurs mesurées tirées du système de mesure du glucose plasmatique et du dispositif de mesure de la glycohémoglobine.
